(19) Europäisches Patentamt
European Patent Office
Office européen des brevets

(11) **EP 2 174 688 B1**

(12) **EUROPÄISCHE PATENTSCHRIFT**

(45) Veröffentlichungstag und Bekanntmachung des Hinweises auf die Patenterteilung:
**28.12.2011 Patentblatt 2011/52**

(51) Int Cl.:
***A61N 1/372*** *(2006.01)*

(21) Anmeldenummer: 09170058.3

(22) Anmeldetag: **11.09.2009**

(54) **Vorrichtung zur Verarbeitung von Herzsignalen**

Device for Processing Cardiac Signals

Dispositif pour traitement des signaux du coeur

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **08.10.2008 DE 102008042681**

(43) Veröffentlichungstag der Anmeldung:
**14.04.2010 Patentblatt 2010/15**

(73) Patentinhaber: **Biotronik CRM Patent AG**
**6341 Baar (CH)**

(72) Erfinder:
• **Busch, Ulrich**
**10318, Berlin (DE)**
• **Neumann, Andreas**
**12437, Berlin (DE)**
• **Tietze, Ulrich**
**13156, Berlin (DE)**

(74) Vertreter: **Lindner-Vogt, Karin L.**
**Biotronik SE & Co. KG**
**Woermannkehre 1**
**12359 Berlin (DE)**

(56) Entgegenhaltungen:
**US-A- 5 514 163       US-A1- 2005 137 632**
**US-A1- 2007 156 194       US-A1- 2008 004 665**

EP 2 174 688 B1

**Beschreibung**

Die Erfindung betrifft eine Vorrichtung zur Verarbeitung von Herzsignalen, und zwar insbesondere ein elektromedizinisches Implantat, wie beispielsweise einen Herzschrittmacher, einen Kardioverter/Defibrillator oder dergleichen.

Derartige Implantate werden in an sich bekannter Weise dazu benutzt, eine zeitlich adäquate Kontraktion der Herzkammern (Ventrikel und/oder Atrium) eines Herzens zu unterstützen oder sicherzustellen. Dazu sind derartige elektromedizinische Implantate in der Regel ausgebildet, natürliche Kontraktionen einer jeweiligen Herzkammer beispielsweise dadurch zu erfassen, dass die mit den natürlichen Kontraktionen einhergehenden elektrischen Potenziale des Myokards der jeweiligen Herzkammer erfasst und ausgewertet werden. Der Verlauf dieser Potenziale ist auch als Elektrokardiogramm bekannt. Die Aufnahme erfolgt dabei jeweils zwischen zwei elektrisch leitfähigen Elektroden, die in geeigneter Weise am oder in der Nähe des Myocards angeordnet sind. Dabei können verschiedene Arten von Elektrokardiogrammen unterschieden werden, je nachdem, wie und wo die Potenzialverläufe erfasst werden. Werden Potenzialverläufe beispielsweise mit einer Vielzahl auf der Haut eines menschlichen Körpers angebrachten Elektroden erfasst, spricht man von einem Oberflächenelektrokardiogramm. Werden die Potenzialverläufe mit Hilfe eines elektronischen Implantats unter Zuhilfenahme von mit diesem verbundenen intrakardial platzierten Elektroden erfasst, spricht man von einem intrakardialen Elektrogramm. Bei den intrakardialen Elektrogrammen können wieder Nahfeldelektrokardiogramme von Fernfeldelektrokardiogrammen unterschieden werden, je nachdem, wie weit jeweils eine der das Potenzial aufnehmenden Elektroden vom Ort der Erregung entfernt ist. Die intrakardiale Aufnahme kann bipolar erfolgen, d.h. zwischen zwei Elektrodenkontakten einer im Atrium oder Ventrikel platzierten Elektrodenleitung. Alternativ oder parallel dazu ist eine unipolare Aufnahme möglich, d.h. zwischen einer im Atrium oder Ventrikel platzierten Elektrode und einer geeigneten Gegenelektrode, welche durch das elektrisch leitfähige Gehäuse gebildet wird. Beispiele für die Bestimmung von Fernfeldelektrokardiogrammen sind in US2008/004665 und US5514163 offenbart.

Durch Aufnahme von Elektrokardiogrammen können in an sich bekannter Weise natürliche (und auch stimulierte) Erregungen des jeweiligen Myokards erfasst werden. Beispielsweise manifestiert sich die Erregung beider Ventrikel in einem markanten Signalabschnitt eines jeweiligen Elektrokardiogramms, der als QRS-Komplex bezeichnet wird. Erregungen des Atriums werden beispielsweise durch Detektion von sogenannten P-Wellen im jeweiligen Elektrokardiogramm detektiert. Auf diese Weise können für die Steuerung eines elektromedizinischen Implantats, insbesondere eines implantierbaren Elektrostimulators, wichtige Ereignisse, einschließlich des Zeitpunkts ihres Auftretens, erfasst werden. Solche Ereignisse können natürliche oder stimulierte atriale Ereignisse sein, also natürliche oder stimulierte Erregungen des Atriums, die zu einer Kontraktion des Atriums führen. Entsprechend können die erfassten Ereignisse auch natürliche oder stimulierte Ereignisse im jeweiligen Ventrikel (rechter Ventrikel oder linker Ventrikel) sein, also eine natürliche oder stimulierte Erregung des jeweiligen Ventrikels mit nachfolgender Kontraktion.

So im Herzen erfasste Ereignisse werden zum einen dazu genutzt, bestimmte Zeitgeber in einem implantierbaren Elektrostimulator zu starten, aber auch, um bestimmte Zeitintervalle zu erfassen und für die Steuerung des Elektrostimulators zu nutzen. Beispielsweise kann durch das Erfassen eines rechtsatrialen natürlichen Ereignisses (also eine Erregung des rechten Atriums) ein Zeitgeber für ein rechtsventrikuläres Escape-Intervall gestartet werden, zu dessen Ende ein rechtsventrikulärer Stimulationsimpuls abgegeben wird, falls vor Ende des rechtsventrikulären Escape-Intervalls nicht eine natürliche Kontraktion des rechten Ventrikels erfasst wird. Bei sogenannten Demand-Schrittmachern wird die Abgabe eines ventrikulären Stimulus dann unterdrückt, wenn während des Verlaufs eines entsprechenden Escape-Intervalls ein natürliches Ereignis in der ansonsten zu stimulierenden Kammer erfasst wird.

Um ein solches rechtsventrikuläres Escape-Intervall möglichst genau an die Bedürfnisse eines individuellen Herzens anzupassen, ist es vorteilhaft, wenn das rechtsventrikuläre Escape-Intervall an eine natürliche atrioventrikuläre Überleitungszeit (AV-Delay) vom rechten Atrium zum rechten Ventrikel angepasst ist und in der Regel nur ein wenig länger als dieses AV-Intervall ist. Ebenso ist es wünschenswert, ein geeignetes Intervall für die zeitliche Verzögerung zwischen der Erregung des linken Atriums und des linken Ventrikels zu bestimmen. Bei der Programmierung dieser zeitlichen Verzögerung ist zu beachten, dass die aktive Kontraktion des linken Ventrikels ca. 100 ms nach der linksatrialen elektrischen Erregung (Ende der linksatrialen P-Welle) erfolgen sollte, da erst nach ca. 100 ms die Füllphase des linken Ventrikels abgeschlossen ist. Der Beginn der linksventrikulären Kontraktion korreliert mit der vollständigen Erregung des Ventrikelmyokards, die im *OberflächenElektrokardiogramm durch eine Spitze des QRS-Komplexes dargestellt ist.

Auch bei einem elektromedizinischen Implantat zur Stimulation des linken Ventrikels soll die entsprechende AV-Zeit des Implantats die mechanische Synchronisation zwischen linkem Atrium und linkem Ventrikel so steuern, dass nicht vor Abschluss der linksatrialen Auswurfphase die Kontraktion des linken Ventrikels beginnt. Die Dauer der linksatrialen Auswurfphase bzw. die aktive Füllungsphase des linken Ventrikels kann als empirisch ermittelter Wert konstant angenommen werden.

Üblicherweise werden für die Steuerung des elektromedizinischen Implantats Elektroden im rechten Atrium bzw. rechten Ventrikel verwendet. Für die Erfassung atrialer Ereignisse mit dieser sogenannten rechtsatrialen Elektrodenlage ergibt sich eine Leitungszeit zwischen dem Erfassen eines rechtsatrialen natürlichen Ereignisses bzw. einer rechtsatrialen Stimulation und dem Beginn der linksatrialen Auswurfphase. Die Dauer dieser Leitungszeit ist allerdings in der Regel nicht zu erfassen. Gleiches gilt auch für die Leitungszeit zwischen einem rechtsventrikulären Stimulus und dem Beginn der linksventrikulären Kontraktion, wenn die Erfassung ventrikulärer Ereignisse mit dieser sogenannten rechtsventrikulären Elektrodenlage erfolgt.

Die durch die Leitungszeiten bedingten Latenzzeiten unterliegen interindividuellen Variationen und müssen deshalb bei jedem Patienten individuell gemessen werden.

Aufgabe der Erfindung ist es, ein elektromedizinisches Implantat und ein Verfahren zu seinem Betreiben anzugeben, welches es erlaubt, eine rechtsatrial gestartete AV-Zeit des Implantats zur rechtsventrikulären Stimulation möglichst automatisch einstellen zu können.

Diese Aufgabe wird durch ein elektromedizinisches Implantat mit einer Fernfeldelektrokardiogramm-Erfassungseinheit gelöst, wobei die Fernfeldelektrokardiogramm-Erfassungseinheit einerseits wenigstens mit einer im rechten Ventrikel oder im rechten Atrium zu platzierenden Elektrode sowie andererseits dem Gehäuse des implantierbaren medizinischen Gerätes als Neutralelektrode verbunden oder zu verbinden ist und ausgebildet ist, über diese Elektroden ein Fernfeldelektrokardiogramm aufzunehmen. Die Fernfeldelektrokardiogramm-Erfassungseinheit ist mit einer Fernfeldelektrokardiogramm-Auswerteeinheit verbunden, die ihrerseits ausgebildet ist, in einem von der Fernfeldelektrokardiogramm-Erfassungseinheit aufgenommenen Fernfeldelektrokardiogramm mit einer Erregung oder Kontraktion eines linken Atriums und/oder eines linken Ventrikels eines Herzens einhergehende Signalmerkmale des Fernfeldelektrokardiogramms zu detektieren.

Der zugrunde liegende Gedanke besteht somit darin, die vollständige elektrische Erregung des Atriums durch geeignete intrakardiale Fernfeld-Ableitungen darzustellen. Punkte, zwischen denen ein solches Fernfeldelektrokardiogramm abgeleitet wird, als die Orte der zur Aufnahme des jeweiligen Fernfeldelektrokardiogramms benutzten Elektroden, sind dabei optimal so gewählt, dass sie den gesamten Erregungsvektor einer jeweiligen Herzkammer überspannen. Beispielsweise kann auf der unipolaren rechtsventrikulären Ableitung die Erregung des linken Vorhofs erkannt werden und entsprechend auf der unipolaren rechtsatrialen Ableitung die Erregung von rechtem und linkem Ventrikel.

Ergebnis ist ein elektromedizinisches Implantat , welches eine rechtsatrial gestartete AV-Zeit des Implantats zur rechtsventrikulären Stimulation möglichst automatisch so einstellt, dass sich zwischen dem Ende der linksatrialen P-Welle und dem Beginn der linksventrikulären Kontraktion genau die Zeit einstellt, die für die linksventrikuläre Füllungsphase benötigt wird.

Dementsprechend ist zum Erfassen linksatrialer Kontraktionen eine Verbindung der Fernfeldelektrokardiogramm-Erfassungseinheit mit einerseits einer im rechten Ventrikel zu platzierenden Elektrode und andererseits mit einer Neutralelektrode in Form eines elektrisch leitenden Gehäuseteils des elektromedizinischen Implantats vorgesehen.

Für das Erfassen linksventrikulärer Kontraktionen ist die Fernfeldelektrokardiogramm-Erfassungseinheit mit einer zur Platzierung im rechten Atrium vorgesehenen Elektrode und andererseits mit einer Neutralelektrode in Form eines elektrisch leitenden Gehäuseteils des elektromedizinischen Implantats verbunden.

Die Fernfeldelektrokardiogramm-Erfassungseinheit ist zum einem mit einer Elektrode zur Platzierung im rechten Ventrikel verbunden oder zu verbinden und zum anderen mit einer elektrisch leitenden Außenoberfläche eines Gehäuses des elektromedizinischen Implantats, wobei die Auswerteinheit dazu ausgebildet ist, in einem derart aufgenommenen Fernfeldelektrokardiogramm mit einer Erregung eines linken Atriums eines Herzens einhergehende Signalmerkmale des Fernfeldelektrokardiogramm zu detektieren und einen AS/AP-Delay-Wert als Zeitdauer zwischen einem erfassten natürlichen oder stimulierten rechtsatrialen Ereignis und einem zugehörigen Ende einer linksatrialen Erregung in dem Fernfeldelektrokardiogramm zu bestimmen. Der AS/AP-Delay-Wert ist hierbei die Zeitdauer zwischen dem Erfassen eines natürlichen (AS) oder alternativ auch eines stimulierten rechtsatrialen (AP) Ereignisses und dem Ende des linksatrialen Ereignisses, also eine Art interatriale Latenzzeit. Zum Erfassen des rechtsatrialen Ereignisses weist das elektromedizinische Implantat vorzugsweise eine an sich bekannte atriale Sensingeinheit auf, die mit einer Sensingelektrode zur Platzierung im rechten Atrium verbunden oder zu verbinden ist.

Zusätzlich ist die Fernfeldelektrokardiogramm-Erfassungseinheit zum einem mit einer Elektrode zur Platzierung im rechten Atrium verbunden oder zu verbinden und zum anderen ist die Fernfeldelektrokardiogramm-Erfassungseinheit mit einer elektrisch leitenden Außenoberfläche eines Gehäuses des elektromedizinischen Implantats verbunden oder zu verbinden, und die Auswerteinheit ist dazu ausgebildet, in einem derart aufgenommenen Fernfeldelektrokardiogramm mit einer Erregung eines linken Ventrikels eines Herzens einhergehende Signalmerkmale des Fernfeldelektrokardiogramm zu detektieren und einen VP-Delay-Wert als Zeitdauer zwischen einem wirksamen rechtsventrikulären Stimulationsimpuls bis zu einem einen Beginn der linksventrikulären Kontraktion in dem Fernfeldelektrokardiogramm anzeigenden Signalmerkmal zu bestimmen. Der VP-Delay-Wert ist somit eine Art interventrikuläre Latenzzeit. Derartige Algorithmen sind aus US2008/004665 und US5514163 bekannt. Gegenstand der Erfindung ist es, den Beginn der linksventrikulären Kontraktion durch das Maximum in dem Fernfeldelektrokardiogramm zu bestimmen.

Außerdem ist ein elektromedizinisches Implantat bevorzugt, das als Elektrostimulator zur elektrischen Stimulation einer oder mehrerer Kammern eines Herzens ausgebildet ist und eine Stimulationssteuereinheit sowie wenigstens einen Stimulationsimpulsgenerator aufweist, die derart miteinander verbunden sind dass der Stimulationsimpulsgenerator durch die Stimulationssteuereinheit gesteuert elektrische Stimulationsimpulse generiert und zu von der Stimulationssteuereinheit bestimmten Zeitpunkten über eine Elektrodenleitung oder einen Anschluss für eine Elektrodenleitung abgibt, wobei die Stimulationssteuereinheit ausgebildet ist anhand des AS/AP-Delays und des VP-Delays eine AV-Zeit für die rechtsventrikuläre Stimulation zu bestimmen.

Die Auswerteinheit kann außerdem dazu ausgebildet sein, das Signal-Rausch-Verhältnis eines linksatrialen Komplexes im aufgenommenen Fernfeldelektrokardiogramm dadurch zu verbessern, dass das Fernfeldelektrokardiogramm über mehrere Herzzyklen gemittelt wird, und zwar vorzugsweise, indem ein jeweiliges natürliches oder stimuliertes atriales Ereignis als Bezugszeitpunkt für die Mittelung genutzt wird, was nicht Teil der Erfindung ist.

Die bereits angesprochene Ermittlung einer optimalen AV-Zeit mit dem Ziel eines daraus mittelbar resultierenden Delays zwischen dem Ende der linksatrialen Erregung und dem Beginn der linksventrikulären Kontraktion nach rechtsventrikulärer Stimulation läuft vorzugsweise wie folgt ab:

1. Zunächst wird die Zeitdauer (AS-Delay-Wert) zwischen dem Erfassen eines natürlichen rechtsatrialen Ereignisses und dem Ende der linksatrialen Erregung in einem Fernfeldelektrokardiogramm bestimmt, welches vorzugsweise mittels einer rechtsventrikulären Elektrode und dem Gehäuse des Implantats als Neutralelektrode gewonnen wurde.

2. Außerdem wird die Zeitdauer (VP-Delay-Wert) von der Abgabe eines rechtsventrikulären Stimulus bis zum Erfassen des Beginns einer linksventrikulären Kontraktion in einem Fernfeldelektrokardiogramm bestimmt, welches vorzugsweise mittels einer rechtsatrialen Elektrode und dem Gehäuse des Implantats als Neutralelektrode gewonnen wurde. Der Zeitpunkt des Erfassens eines Maximums der ventrikulären Gesamterregung wird als Endzeitpunkt der zu bestimmenden Zeitdauer erfasst.

3. Eine durchschnittliche aktive Füllungsphase (Füll-Delay) wird beispielsweise auf 100ms festgelegt, also auf einen empirisch ermittelten Wert;

4. Die zu bestimmende AV-Zeit nach Erfassen eines natürlichen rechtsatrialen Ereignisses berechnet sich dann wie folgt:

$$\text{AV Zeit} = \text{AS-Delay} + \text{Füll-Delay} - \text{VP-Delay}$$

Die Ermittlung einer optimalen AV-Zeit-Einstellung nach Abgabe eines rechtsatrialen Stimulus kann analog zur Ermittlung der AV-Zeit nach einem rechtsatrialen Ereignis erfolgen, nur getriggert durch den atrialen Stimulus (AP-Delay).

Das Verfahren erlaubt die automatische Einstellung der AV-Zeit nach A-Sense bzw. A-Pace durch Erfassung der interatrialen und interventrikulären Latenzzeiten des Myokards.

Dieses Verfahren ist für alle Zwei- und Dreikammer-Implantate anwendbar.

Weitere vorteilhafte Ausgestaltungen ergeben sich durch Kombination der hier beschriebenen Merkmale untereinander und mit solchen Merkmalen, die aus dem Stand der Technik bekannt sind.

Die Erfindung soll nun anhand eines Ausführungsbeispiels unter Bezug auf die Figuren näher erläutert werden. Diese zeigen in

Figur 1:  eine Darstellung eines biventrikulären Herzstimulators mit angeschlossenen, im Herzen angeordneten Elektroden; und

Figur 2:  ein schematisches Blockschaltbild eines Herzstimulators.

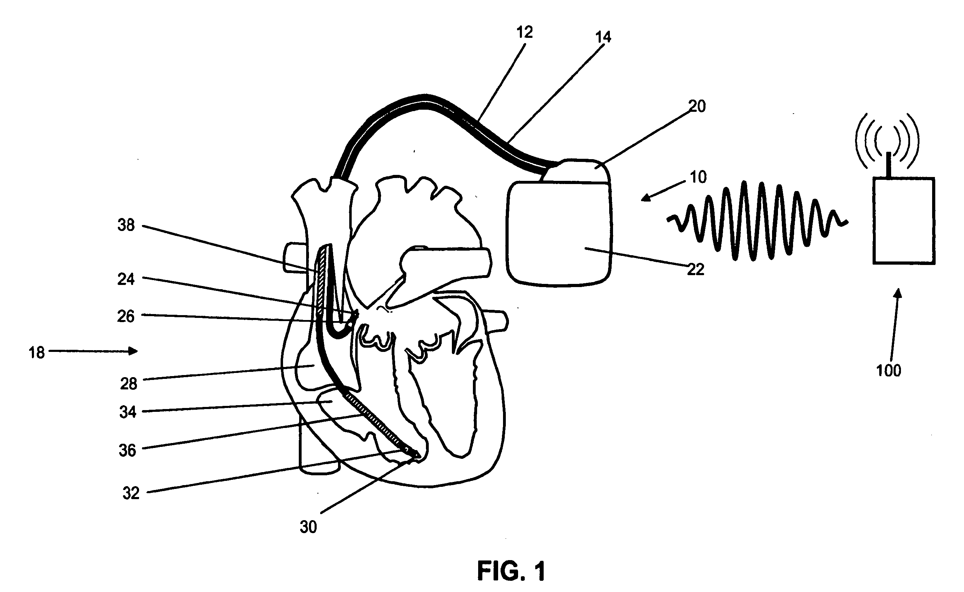

Figur 1 zeigt einen implantierbaren Herzstimulator 10 in Form eines Zweikammerherzschrittmachers/Kardioverter/Defibrillators mit daran angeschlossenen Elektrodenleitungen 12 und 14 in Verbindung mit einem Herz 18. Außerdem ist ein externes Gerät 100 in der Nähe des implantierbaren Herzstimulators 10 dargestellt. Die Elektrodenleitungen 12 und 14 sind über bekannte, standardisierte Steckverbindungen mit Kontaktbuchsen in einem Header (Anschlussgehäuse) 20 des Herzstimulators 10 elektrisch verbunden. Auf diese Weise sind die Elektrodenleitungen 12 und 14 auch mit elektronischen Komponenten im inneren eines hermetisch dichten Metallgehäuses 22 des Herzstimulators 10 verbunden. Diese Komponenten sind nachfolgend detaillierter schematisch dargestellt und bestimmen die erfindungsgemäße Funktionsweise des Herzstimulators 10.

Die Elektrodenleitung 12 ist eine rechtsatriale Elektrodenleitung und besitzt an ihrem distalen Ende eine atriale Spitzenelektrode RA Tip 24 sowie in kurzer Entfernung davon eine atriale Ringelektrode RA Ring 26, die beide im rechten Atrium 28 des Herzens 18 platziert sind.

Die Elektrodenleitung 14 ist eine rechtsventrikuläre Elektrodenleitung und besitzt an ihrem distalen Ende eine rechtsventrikuläre Spitzenelektrode RV Tip 30 und in unmittelbarer Nähe eine rechtsventrikuläre Ringelektrode RV Ring 32. Beide Elektroden sind im Apex des rechten Ventrikels 34 des Herzens 18 angeordnet.

Außerdem hat die rechtsventrikuläre Elektrodenleitung 14 noch eine rechtsventrikuläre Schockwendel RV Schock 36 als großflächige Elektrode zur Abgabe von Defibrillationsschocks. Eine weitere Schockwendel 38 ist in der Vena Cava Superior angeordnet und wird deshalb nachfolgend auch als SVC-Schockelektrode bezeichnet.

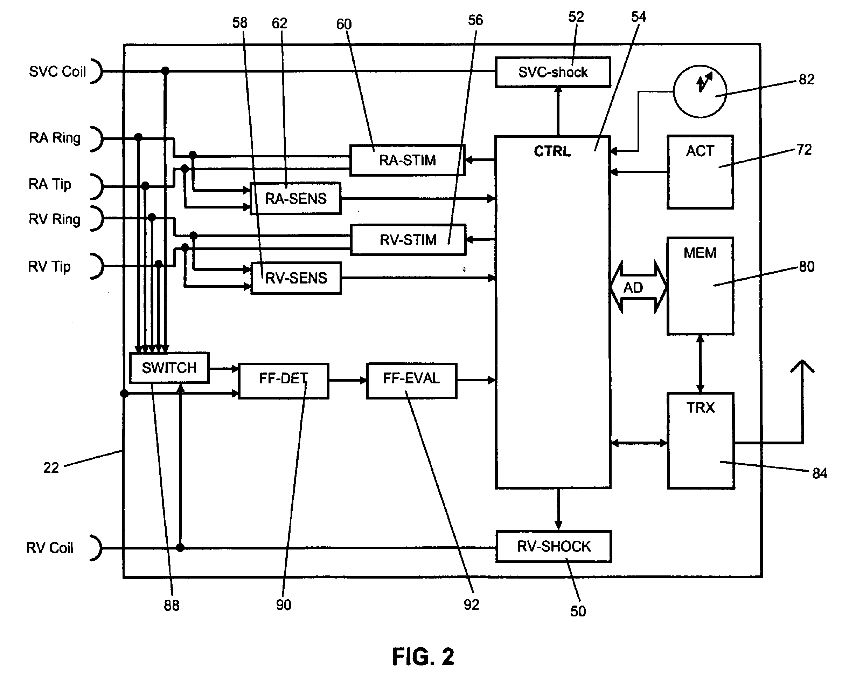

In Figur 2 sind die Hauptbestandteile des Herzstimulators 10 dargestellt. Auf der linken Seite sind die elektrischen Anschlüsse für die verschiedenen Elektroden 24, 26, 30, 32, 36 und 38 dargestellt. Die Schockelektroden 36 und 38 sind jeweils mit einem rechtsventrikulären Schockimpulsgenerator 50 bzw. SVC-Schockgenerator 52 verbunden. Beide Schockgeneratoren 50 und 52 sind mit einer Stimulationssteuereinheit 54 verbunden, die die beiden Schockimpulsgeneratoren 50 und 52 bei Bedarf zur Erzeugung und Abgabe eines Defibrillationsschocks ansteuert.

Der Anschluss für die rechtsventrikuläre Spitzenelektrode RV Tip sowie der Anschluss für die rechtsventrikuläre Ringelektrode RV Ring sind jeweils sowohl mit einer rechtsventrikulären Stimulationseinheit 56 als auch mit einer rechtsventrikulären Sensingeinheit 58 verbunden. Sowohl die rechtsventrikuläre Stimulationseinheit 56 als auch die rechtsventrikuläre Sensingeinheit 58 sind jeweils mit der Stimulationssteuereinheit 54 verbunden.

Die rechtsventrikuläre Stimulationseinheit 56 ist dazu ausgebildet, auf ein Ansteuersignal der Stimulationssteuereinheit 54 hin einen rechtsventrikulären Stimulationsimpuls zu erzeugen und über die Anschlüsse der rechtsventrikulären Ringelektrode RV Ring und der rechtsventrikulären Spitzenelektrode RV Tip abzugeben. Alternativ ist es auch möglich, dass das Gehäuse 22 des Herzstimulators 10 eine neutrale Elektrode bildet und die rechtsventrikuläre Stimulationseinheit 56 mit dem Anschluss für die rechtsventrikuläre Spitzenelektrode RV Tip und dem Gehäuse 22 als andere Elektrode zur Abgabe eines Stimulationsimpulses verbunden ist. Ein rechtsventrikulärer Stimulationsimpuls unterscheidet sich von einem Defibrillationsschock dadurch, dass der Stimulationsimpuls eine wesentlich geringere Impulsstärke besitzt, so dass er nicht wie ein Defibrillationsschock auf einen Schlag das vollständige Herzgewebe (Myokard) einer Herzkammer erregt, sondern nur die Herzmuskelzellen in unmittelbarer Umgebung der rechtsventrikulären Spitzenelektrode RV Tip 30. Diese Erregung breitet sich dann durch natürliche Reizleitung über den gesamten rechten Ventrikel 34 weiter aus und sorgt so für eine stimulierte Kontraktion des rechten Ventrikels 34.

Die rechtsventrikuläre Sensingeinheit 58 ist dazu ausgebildet, an dem Anschluss für die rechtsventrikuläre Ringelektrode RV Ring und die rechtsventrikuläre Tipelektrode RV Tip anliegende elektrische Potentiale zunächst durch einen Eingangsverstärker zu verstärken und zu filtern. Weiterhin ist die rechtsventrikuläre Sensingeinheit 58 ausgebildet, den Verlauf der an ihren Eingängen anliegenden elektrischen Signale derart auszuwerten, dass die rechtsventrikuläre Sensingeinheit 58 selbsttätig eine natürliche (intrinsische), d. h. selbsttätige Kontraktion des rechten Ventrikels 34 detektiert. Dies kann beispielsweise dadurch geschehen, dass der Verlauf des an den Eingängen der rechtsventrikulären Sensingeinheit 58 anliegenden Signals mit einem Schwellwert verglichen wird. Typischerweise ist die größte Amplitude des Signals in Form der sogenannten R-Zacke kennzeichnend für eine natürliche Kontraktion des rechten Ventrikels 34, die durch Schwellwertvergleich detektiert werden kann. Die rechtsventrikuläre Sensingeinheit 58 gibt daraufhin ein entsprechendes, eine natürliche Kontraktion des rechten Ventrikels 34 anzeigendes Ausgangssignal an die Stimulationssteuereinheit 54 aus.

In analoger Weise sind der Anschluss für die rechtsatriale Spitzenelektrode RA Tip und der Anschluss für die rechtsatriale Ringelektrode RA Ring sowohl mit einer rechtsatrialen Stimulationseinheit 60 als auch mit einer rechtsatrialen Sensingeinheit 62 verbunden, die jeweils ihrerseits wiederum mit der Stimulationssteuereinheit 54 verbunden sind. Die rechtsatriale Stimulationseinheit 60 ist dazu ausgebildet, Stimulationsimpulse zu erzeugen, deren Stärke ausreicht, das rechtsatriale Myokard zu erregen. Die rechtsatrialen Stimulationsimpulse können dabei eine andere Impulsstärke besitzen, als die rechtsventrikulären Stimulationsimpulse. Die rechtsatriale Sensingeinheit 62 ist ausgebildet, aus dem Verlauf des an ihren Eingängen anliegenden Differenzsignals eine sogenannte P-Welle zu detektieren, die eine natürliche (intrinsische) Kontraktion des rechten Atriums 28 kennzeichnet. Detektiert die rechtsatriale Sensingeinheit 62 eine entsprechende P-Welle, erzeugt sie ein Ausgangssignal und gibt dieses an die Stimulationssteuereinheit 54 weiter, welches eine natürliche Kontraktion des rechten Atriums 28 kennzeichnet.

Als weiterer Bestandteil des Herzstimulators 10 ist ein Beschleunigungssensor 72 mit der Stimulationssteuereinheit 54 verbunden und in das Gehäuse 22 des Herzstimulators 10 integriert. Der Beschleunigungssensor 72 ist dazu ausgebildet, ein von der körperlichen Aktivität eines Patienten abhängiges Bewegungssignal zu erfassen und ein entsprechendes, die körperliche Aktivität des Patienten anzeigendes erstes Akzelerometer-Ausgangssignal an die Stimulationssteuereinheit 54 auszugeben. Dies erlaubt es, dass die Stimulationssteuereinheit 54 das Timing der Stimulationsimpulse an den Bedarf des Patienten (hämodynamischen Bedarf) anpasst.

Weiterhin umfasst der Herzstimulator 10 eine Speichereinheit 80, die mit der Stimulationssteuereinheit 54 verbunden ist und es erlaubt, von der Stimulationssteuereinheit 54 erzeugte oder ausgewertete Signale zu speichern. Andererseits erlaubt es die Speichereinheit 80, Steuerprogramme für die Stimulationssteuereinheit 54 in veränderbarer Form zu speichern. Des weiteren ist die Stimulationssteuereinheit 54 mit einem Zeitgeber 82 verbunden.

Die Speichereinheit 80 ist mit einer Telemetrieeinheit 84 verbunden, die es erlaubt, in der Speichereinheit 80 abgelegte Daten drahtlos an das externe Gerät 100 zu übertragen oder Programmierbefehle seitens des externen Geräts 100 zu dem Herzstimulator 10 zu übertragen und in der Speichereinheit 80 zu speichern.

Als Zweikammerherzstimulator/Kardioverter/Defibrillator ist der Herzstimulator 10 in der Lage, eine Stimulation des rechten Atriums 28, des rechten Ventrikels 34 und des linken Ventrikels 44 oder auch nur einer oder zweier dieser Herzkammern in an sich bekannter Weise durchzuführen. Dies schließt insbesondere die Stimulation einer jeweiligen Herzkammer im Demand-Modus ein, in der Stimulationsimpulse nur dann an die jeweilige Herzkammer abgegeben werden, falls in einem vorangehenden, jeweiligen Escape-Intervall seitens der jeweiligen Sensingeinheit keine intrinsische Kontraktion der jeweiligen Herzkammer erfasst wird. Damit ist der Herzstimulator 10 in der Lage, die bekannten rechtsventrikulären Stimulationsmodi wie VVI, VVD oder DDD durchzuführen.

Für das Timing der Stimulationsimpulse im rechtsventrikulären Stimulationsmodus wird idealerweise auch die Verzögerungszeit berücksichtigt, mit der eine linksventrikuläre Kontraktion auf eine rechtsventrikuläre Stimulation folgt (VP-Delay).

Um die Implantat-bedingten interatrialen Zeitintervalle für VDD- und DDD-Simulation zu bestimmen, besitzt der Herzstimulator 10 erfindungsgemäß eine Fernfeldelektrokardiogramm-Erfassungseinheit 90. Diese kann einerseits wahlweise mit einer rechtsventrikulären Elektrode oder einer rechtsatrialen Elektrode sowie andererseits mit dem Schrittmachergehäuse 22 als Neutralelektrode verbunden werden, so dass das erfasste und verarbeitete Potential ein unipolares Herzpotential ist. Zur Auswahl einer geeigneten Elektrodenkonfiguration für die Aufnahme eines Fernfeldelektrokardiogramms ist eine Schalteinheit 88 vorgesehen.

Mit dem Ausgang der Fernfeldelektrokardiogramm Erfassungseinheit 90 ist eine Auswerteeinheit 92 verbunden. Die Auswerteeinheit 92 ist ausgebildet, ein jeweiliges von der Fernfeldelektrokardiogramm-Erfassungseinheit 90 erfasstes Fernfeldelektrokardiogramm auszuwerten und für linksatriale bzw. linksventrikuläre Erregungen charakteristische Signalmerkmale zu detektieren. Signalmerkmale, die für linksatriale Erregungen charakteristisch sind, werden in einem Fernfeldelektrokardiogramm detektiert, die das über eine rechtsventrikuläre Elektrode und das Gehäuse 22 des Implantats aufgenommen wurde und Signalmerkmale, die für linksventrikuläre Erregungen charakteristisch sind, werden in einem Fernfeldelektrokardiogramm detektiert, die das über eine rechtsatriale Elektrode und das Gehäuse 22 des Implantats aufgenommen wurde.

Dementsprechend liefert die Auswerteeinheit 92 unterschiedliche Ausgangssignale (Marker) für linksventrikuläre und linksatriale Ereignisse. Die Ausgangssignale (Marker) für linksatriale Ereignisse werden dabei für einmalige oder zyklische Bestimmung der Dauer interatrialen Leitungszeiten genutzt.

Zu diesem Zweck kann die Auswerteeinheit 92 ausgebildet sein, eine jeweilige linksventrikuläre Kontraktion und eine jeweilige linksatriale Kontraktion kennzeichnende Signalmerkmale anhand eines Vergleich von Morphologiemerkmalen eines jeweils aktuellen Signals mit gespeicherten Morphologiemerkmalen durchzuführen, was nicht Teil der Erfindung ist. Dazu unterzieht die Auswerteeinheit 92 das jeweilige mittels Fernfeldelektrokardiogramm-Erfassungseinheit 90 über die rechtsventrikuläre Elektrode oder die rechtsatriale aufgenommene Fernfeldelektrokardiogramm einer Wavelettransformation und vergleicht anschließend so gewonnene Waveletkoeffizienten mit in einem Speicher 80 gespeicherten Vergleichskoeffizienten, um so gegebenenfalls eine jeweilige linksatriale bzw. linksventrikuläre Kontraktion zu detektieren.

Auch kann die Auswerteeinheit 92 so ausgebildet sein, dass die Zeitpunkte des Auftretens der oben genannten Morphologiemerkmale innerhalb des Stimulationszyklus erst nach Aufsummierung einer bestimmten Anzahl einzelner Herzaktionen im Sinne einer Signalmittelungstechnik bestimmt werden.

Die Bestimmung der Dauer der linksseitigen AV-Zeit kann auch mit dem Programmiergerät erfolgen. Hierzu sind die entsprechende Signalmerkmale enthaltenden Elektrogramme oder Marker telemetrisch vom Schrittmacher in das Programmiergerät zu übertragen. Die Erfassungseinheit ist dann Bestandteil des Programmiergerätes. Die Messungen erfolgen dann entweder automatisch im Programmiergerät oder manuell, indem die Elektrogramme oder Marker am Programmerbildschirm dargestellt, eingefroren und mittels Callippern vermessen werden.

Die Auswerteeinheit kann auch auf das externe Gerät 100 ausgelagert sein. Dann ist der Herzstimulator 10 ausgebildet, ein jeweiliges von der Fernfeldelektrokardiogramm-Erfassungseinheit 90 aufgenommenes Fernfeldelektrokardiogramm beschreibende Daten mittels einer Telemetrieeinheit 84 telemetrisch auf das externe Gerät 100 zu übertragen. Beides ist nicht Teil der Erfindung.

Die Auswerteeinheit 92 ist zur Bestimmung eines AV-Delays zwischen rechtem Atrium und rechtem Ventrikel unter Wahrung einer zeitlich optimalen linksseitigen AV-Sequenz derart ausgebildet, dass die Auswerteeinheit:

- die Zeitdauer (AS-Delay-Wert) zwischen dem Erfassen eines natürlichen rechtsatrialen Ereignisses und dem Ende der linksatrialen Erregung in einem Fernfeldelektrokardiogramm bestimmt, welches vorzugsweise mittels einer rechtsventrikulären Elektrode und dem Gehäuse des Implantats als Neutralelektrode gewonnen wurde.

- die Zeitdauer (VP-Delay-Wert) von der Abgabe eines rechtsventrikulären Stimulus bis zum Erfassen des Beginns einer *linksventrikulären Kontraktion in einem Fernfeldelektrokardiogramm bestimmt, welches vorzugsweise mittels einer rechtsatrialen Elektrode und dem Gehäuse des Implantats als Neutralelektrode gewonnen wurde.

- eine durchschnittliche aktive Füllungsphase (Füll-Delay) beispielsweise auf einen empirisch ermittelten Wert festgelegt; und

- die zu bestimmende AV-Zeit nach Erfassen eines natürlichen rechtsatrialen Ereignisses nach folgender Formel berechnet:

**AV Zeit = AS-Delay + Füll-Delay – VP-Delay**

Der Beginn einer linksventrikulären Kontraktion in dem Fernfeldelektrokardiogramm als Endzeitpunkt der zu bestimmenden Zeitdauer (VP-Delay) wird durch den Zeitpunkt des Erfassens eines Maximums der ventrikulären Gesamterregung bestimmt.

Ein geeigneter Wert für die durchschnittliche aktive Füllungsphase (Füll-Delay) ist 100ms.

Die Ermittlung einer optimalen AV-Zeit-Einstellung nach Abgabe eines rechtsatrialen Stimulus kann analog zur Ermittlung der AV-Zeit nach einem rechtsatrialen Ereignis erfolgen, nur getriggert durch den atrialen Stimulus (AP-Delay).

Wie in Figur 2 beispielhaft dargestellt, ist die AVD-Bestimmungseinheit 92 außerdem ausgebildet, die rechtsventrikuläre Stimulationseinheit 56 zum Ende eines AV Intervalls auszulösen, falls sie nicht zuvor durch ein von der rechtsventrikulären Sensingeinheit 58 erfasstes rechtsventrikuläres Senseereignis zurückgesetzt wird, so dass ventrikuläre Stimuli nur bei Bedarf abgegeben werden. Dieses AV Intervall entspricht vorzugsweise der zuvor ermittelten AV-Zeit zuzüglich eines Hystereseintervalls.

Bezugszeichenliste

| Bezugszeichen | Bedeutung |
|---|---|
| 10 | Herzstimulator |
| 100 | externes Gerät |
| 12 | rechtsatriale Elektrodenleitung |
| 14 | rechtsventrikuläre Elektrodenleitung |
| 18 | Herz |
| 20 | Header (Anschlussgehäuse) |
| 22 | Gehäuse |
| 24 | atriale Spitzenelektrode RA Tip |
| 26 | atriale Ringelektrode RA Ring |
| 28 | rechtes Atrium |
| 30 | rechtsventrikuläre Spitzenelektrode RV Tip |
| 32 | rechtsventrikuläre Ringelektrode RV Ring |
| 34 | rechter Ventrikel |
| 36 | rechtsventrikuläre Schockwendel RV Schock |
| 38 | Schockwendel (SVG-Schockelektrode) |
| 50 | rechtsventrikulärer Schockimpulsgenerator |
| 52 | SVC-Schockimpulsgenerator |
| 54 | Stimulationssteuereinheit |
| 56 | rechtsventrikuläre Stimulationseinheit |
| 58 | rechtsventrikuläre Sensingeinheit |
| 60 | rechtsatriale Stimulationseinheit |
| 62 | rechtsatriale Sensingeinheit |
| 72 | Beschleunigungssensor |
| 80 | Speichereinheit |
| 82 | Zeitgeber |
| 84 | Telemetrieeinheit |

(fortgesetzt)

| Bezugszeichen | Bedeutung |
|---|---|
| 88 | Schalteinheit |
| 90 | Fernfeldelektrokardiogramm-Erfassungseinheit |
| 92 | Auswerteeinheit |

**Patentansprüche**

1. Elektromedizinisches Implantat (10) mit einem Gehäuse (22) mit einer wenigstens in Teilen elektrisch leitenden Außenoberfläche und einer Fernfeldelektrokardiogramm-Erfassungseinheit (90), die mit wenigstens zwei implantierbaren Elektroden zu verbinden ist, von denen eine Elektrode eine im rechten Atrium zu platzierende Elektrode (24, 26) ist und eine weitere Elektrode eine im rechten Ventrikel zu platzierende Elektrode (30, 32) ist, wobei die Fernfeldelektrokardiogramm-Erfassungseinheit (90) ausgebildet ist, über den Anschluss für die im rechten Atrium oder rechten Ventrikel zu platzierende Elektrode und einer durch das Gehäuse (22) gebildeten, weiteren Elektrode ein Fernfeldelektrokardiogramm aufzunehmen, wobei die Fernfeldelektrokardiogramm-Erfassungseinheit (90) mit einer Auswerteeinheit (92) verbunden ist, die ausgebildet ist, in einem von der Fernfeldelektrokardiogramm-Erfassungseinheit (90) aufgenommenen Fernfeldelektrokardiogramm mit einer Erregung oder Kontraktion eines linken Atriums und/oder eines linken Ventrikels eines Herzens einhergehende Signalmerkmale des Fernfeldelektrokardiogramms zu detektieren, und die Auswerteeinheit (92) zur Ermittlung einer optimalen AV-Zeit für rechtsventrikuläre Stimulation derart ausgebildet ist, dass die Auswerteeinheit (92):

   - die Zeitdauer (AS-Delay-Wert) zwischen dem Erfassen eines natürlichen rechtsatrialen Ereignisses und dem Ende der linksatrialen Erregung in einem Fernfeldelektrokardiogramm bestimmt, welches von der im rechten Ventrikel zu platzierenden Elektrode (30, 32) und dem Gehäuse (22) aufgenommen wird,
   - die Zeitdauer (VP-Delay-Wert) von der Abgabe eines rechtsventrikulären Stimulus bis zum Erfassen des Beginns einer linksventrikulären Kontraktion in einem Fernfeldelektrokardiogramm bestimmt, welches von der im rechten Atrium zu platzierenden Elektrode (24, 26) und dem Gehäuse (22) aufgenommen wird,
   - eine durchschnittliche aktive Füllungsphase (Füll-Delay) auf einen empirisch ermittelten Wert festgelegt; und
   - die zu bestimmende AV-Zeit nach Erfassen eines natürlichen rechtsatrialen Ereignisses nach folgender Formel berechnet:

$$\text{AV Zeit} = \text{AS-Delay} + \text{Füll-Delay} - \text{VP-Delay}$$

   **dadurch gekennzeichnet, dass** die Zeitdauer (VP-Delay-Wert) als die Zeitdauer von der Abgabe eines rechtsventrikulären Stimulus bis zum Erfassen eines Maximums in dem ventrikulären Gesamtkomplex in einem Fernfeldelektrokardiogramm bestimmt wird.

2. Elektromedizinisches Implantat nach Anspruch 1, **dadurch gekennzeichnet, dass** das elektromedizinisches Implantat (10) als Elektrostimulator zur elektrischen Stimulation einer oder mehrerer Kammern eines Herzens ausgebildet ist und eine Stimulationssteuereinheit (54) sowie wenigstens einen Stimulationsimpulsgenerator (64) aufweist, die derart miteinander verbunden sind dass der Stimulationsimpulsgenerator (64) durch die Stimulationssteuereinheit (54) gesteuert elektrische Stimulationsimpulse generiert und zu von der Stimulationssteuereinheit (54) bestimmten Zeitpunkten über eine Elektrodenleitung (16) oder einen Anschluss für eine Elektrodenleitung abgibt, wobei die Stimulationssteuereinheit (54) ausgebildet ist anhand detektierter linksatrialer Kontraktionen eine atrioventrikuläre Verzögerungszeit (AV-Zeit) für die Bestimmung des Zeitpunktes der Abgabe eines rechtsventrikulären Stimulationsimpulses in Abhängigkeit der Zeitpunkte detektierter rechtsatrialer Kontraktionen zu bestimmen.

3. Elektromedizinisches Implantat nach einem der Ansprüche 1 bis 2, **dadurch gekennzeichnet, dass** die Auswerteeinheit (92) ausgebildet ist, die zu bestimmende AV-Zeit nach Erfassen eines stimulierten rechtsatrialen Ereignisses nach folgender Formel zu bestimmen:

$$\text{AV Zeit} = \text{AP-Delay} + \text{Füll-Delay} - \text{VP-Delay}$$

wobei AP-Delay die Zeitdauer zwischen der Abgabe eines rechtsatrialen Stimulationsimpulses und dem Ende der linksatrialen Erregung in einem Fernfeldelektrokardiogramm ist.

**4.** Elektromedizinisches Implantat nach einem der Ansprüche 1 bis 3, **dadurch gekennzeichnet, dass** der empirisch ermittelte Wert für durchschnittliche aktive Füllungsphase (Füll-Delay) auf 100 ms festgelegt wird.

**Claims**

**1.** An electromedical implant (10), comprising a housing (22) having an at least regionally conductive outer surface and a far field electrocardiogram detection unit (90), which is to be connected to at least two implantable electrodes, of which one electrode is an electrode (24, 26) to be placed in the right atrium and a further electrode is an electrode (30, 32) to be placed in the right ventricle, the far field electrocardiogram detection unit (90) being designed to record a far field electrocardiogram via the connection for the electrode to be placed in the right atrium or right ventricle and a further electrode formed by the housing (22), the far field electrocardiogram detection unit (90) being connected to an evaluation unit (92), which is designed to detect signal characteristics of the far field electrocardiogram which accompany an excitation or contraction of the left atrium and/or the left ventricle of a heart in a far field electrocardiogram recorded by the far field electrocardiogram detection unit (90), and the evaluation unit (92) for determining an optimal AV period for right-ventricular stimulation being designed so that the evaluation unit (92):

- determines the duration (AS delay value) between the time a natural right atrial event is detected and the left atrial excitation ends in a far field electrocardiogram, which is recorded by the electrode (30, 32) to be placed in the right ventricle and the housing (22),
- determines the duration (VP delay value) from the time a right-ventricular stimulation is delivered until the start of a left-ventricular contraction is detected in a far field electrocardiogram, which is recorded by the electrode (24, 26) to be placed in the right atrium and the housing (22),
- establishes an average active filling phase (fill delay) to an empirically determined valued; and
- calculates the AV period to be determined upon detection of a natural right atrial event according to the following formula:

$$AV\ Period = AS\ Delay + Fill\ Delay - VP\ Delay$$

**characterized in that** the duration (VP delay value) is determined as the duration from the time a right-ventricular stimulation is delivered until a maximum is detected in the overall ventricular complex in a far field electrocardiogram.

**2.** The electromedical implant according to claim 1, **characterized in that** the electromedical implant (10) is designed as an electrostimulator for electrically stimulating one or more chambers of a heart and comprises a stimulation control unit (54) and at least one stimulation pulse generator (64), which are connected to each other so that the stimulation pulse generator (64) generates electric stimulation pulses in a manner controlled by the stimulation control unit (54) and delivers them at times determined by the stimulation control unit (54) via an electrode line (16) or a connection for an electrode line, wherein the stimulation control unit (54) is designed to determine, based on detected left atrial contractions, an atrioventricular delay (AV period) for determining the time of delivering a right-ventricular stimulation pulse as a function of the times of detected right atrial contractions.

**3.** An electromedical implant according to any one of claims 1 to 2, **characterized in that** the evaluation unit (92) is designed to determine the AV period to be determined upon detection of a stimulated right atrial event according to the following formula:

$$AV\ Period = AP\ Delay + Fill\ Delay - VP\ Delay$$

wherein the AP delay is the duration between the time a right atrial stimulation pulse is delivered and the left atrial excitation ends in a far field electrocardiogram.

**4.** An electromedical implant according to any one of claims 1 to 3, **characterized in that** the empirically determined value for an average active fill phase (fill delay) is established at 100 ms.

**Revendications**

**1.** Implant électro-médical (10) avec un boîtier (22) avec une surface extérieure au moins partiellement électriquement conductrice et une unité de détection d'électrocardiogramme de champ lointain (90), qui est destinée à être reliée à au moins deux électrodes implantables, parmi lesquelles une électrode est une électrode (24, 26) destinée à être placée dans l'oreillette droite et une autre électrode (30, 32) est destinée à être placée dans le ventricule droit, dans lequel l'unité de détection d'électrocardiogramme de champ lointain (90) est conçue pour enregistrer un électrocardiogramme de champ lointain par le raccordement pour l'électrode destinée à être placée dans l'oreillette droite ou dans le ventricule droit et par une autre électrode formée par le boîtier (22), dans lequel l'unité de détection d'électrocardiogramme de champ lointain (90) est reliée à une unité d'évaluation (92) conçue pour détecter les symboles de signaux d'électrocardiogramme de champ lointain qui accompagnent l'excitation ou la contraction de l'oreillette gauche et/ou du ventricule gauche d'un coeur, dans un électrocardiogramme de champ lointain enregistré par l'unité de détection d'électrocardiogramme de champ lointain (90), et dans lequel l'unité d'évaluation (92) pour la détermination d'une durée AV optimale pour la stimulation du ventricule droit est conçue de manière à ce que l'unité d'évaluation (92) :

- définit la durée (valeur AS-Delay) entre la détection d'un événement naturel de l'oreillette droite et la fin d'une excitation de l'oreillette gauche, dans un électrocardiogramme de champ lointain enregistré par l'électrode (30, 32) destinée à être placée dans le ventricule droit et par le boîtier (22),
- définit la durée (valeur VP Delay) de l'indication d'un stimulus du ventricule droit jusqu'à la détection du début d'une contraction du ventricule gauche, dans un électrocardiogramme de champ lointain enregistré par l'électrode (24, 26) destinée à être placée dans l'oreillette droite et par le boîtier (22),
- fixe une phase de remplissage active moyenne (Delay de remplissage) à une valeur déterminée empiriquement ; et
- calcule la durée AV à définir, après la détection d'un évènement naturel de l'oreillette droite, d'après la formule suivante :

$$\text{Durée AV} = \text{Delay AS} + \text{Delay de remplissage} - \text{Delay VP}$$

**caractérisé en ce que** la durée (valeur Delay VP) est déterminée comme étant la durée de l'indication d'un stimulus du ventricule droit, jusqu'à la détection d'un maximum dans le complexe ventriculaire global, dans un électrocardiogramme de champ lointain.

**2.** Implant électro-médical selon la revendication 1, **caractérisé en ce que** l'implant électro-médical (10) est conçu comme un électro-stimulateur pour la stimulation électrique d'une ou de plusieurs chambres d'un coeur, et comporte une unité de commande de stimulation (54) ainsi qu'au moins un générateur d'impulsions de stimulation (64), qui sont reliés l'un à l'autre de manière à ce que le générateur d'impulsions de stimulation (64) génère des impulsions de stimulation électriques commandées par l'unité de commande de stimulation (54) et les transmet, à des moments déterminés par l'unité de commande de stimulation (54), par le biais d'une ligne d'électrodes (16) ou d'un raccord pour une ligne d'électrodes, dans lequel l'unité de commande de stimulation (54) est conçue pour déterminer un temps de retard (durée AV) atrio-ventriculaire à l'aide de contractions détectées de l'oreillette gauche, pour la détermination d'un moment de délivrance d'impulsions de stimulation du ventricule droit, en fonction des moments de contractions détectées de l'oreillette droite.

**3.** Implant électro-médical selon l'une des revendications 1 à 2, **caractérisé en ce que** l'unité d'évaluation (92) est conçue pour déterminer la durée AV à déterminer, après la détection d'un événement stimulé de l'oreillette droite, d'après la formule suivante :

$$\text{Durée AV} = \text{Delay AP} + \text{Delay de remplissage} - \text{Delay VP}$$

où le Delay AP correspond à la durée entre la délivrance d'une impulsion de stimulation de l'oreillette droite et la fin de l'excitation de l'oreillette gauche dans un électrocardiogramme de champ lointain.

4. Implant électro-médical selon l'une des revendications 1 à 3, **caractérisé en ce que** la valeur déterminée empiriquement pour la phase de remplissage active moyenne (Delay de remplissage) est fixée à 100 ms.

**FIG. 1**

EP 2 174 688 B1

FIG. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2008004665 A
- US 5514163 A